# EUROPÄISCHE PATENTANMELDUNG

(11) **EP 1 252 869 A1**
(43) Veröffentlichungstag der Anmeldung: **30.10.2002**
(21) Anmeldenummer: 01110260.5
(22) Anmeldetag: 25.04.2001
(51) Int. Cl.: A61F 2/38

(54) **Knieprothese mit Rotationslager**

(71) Anmelder: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(57) **Zusammenfassung**

Knieprothese, bestehend aus einer Femurkomponente (1), einer Tibiakomponente (3) und einem diese verbindenden Zwischenstück (10), das mit der Femurkomponente (1) ein Beugelager (11) und mit der Tibiakomponente (3) ein Rotationslager (14, 15) bildet, das gegenüber dem Tibiateil (3) verriegelbar ist. Um dem Arzt zu erlauben, während der Operation zu entscheiden, ob eine rotationsfreie oder rotationsfeste Prothese verwendet wird, ist vorgesehen, daß das Zwischenstück (10) wahlweise mit einem Vorsprung versehen ist, der in eine passende Ausnehmung in der Tibiakomponente (4) eingreift.

## Beschreibung

Je schlechter der Erhaltungszustand des Bandapparats eines mit einer Knieprothese zu versorgenden Knies ist, um so weniger inhärente Stabilität wird von der Prothese verlangt. Hat man es mit einem Bandapparat verringerter Stabilität zu tun, kann es notwendig sein, einen Prothesentyp zu verwenden, bei dem die Relativbewegung zwischen Femurkomponente und Tibiakomponente durch einen stabilisierenden Zwischenteil beschränkt ist auf die Beugebewegung um eine horizontale Querachse und eine Rotationsbewegung um eine im wesentlichen tibiaparallele Achse (DE-C-2 744 710). Dafür ist erforderlich, daß der Bandapparat wenigstens noch die Rotationsbewegungen kontrollieren kann. Ist auch diese Fähigkeit nicht erhalten, muß die Rotationsbewegung ausgeschlossen und der Freiheitsgrad der Prothese auf die Beugebewegung beschränkt werden. Üblicherweise verwendet man für diese beiden Anwendungsfälle unterschiedliche Prothesenbauarten. Wenn erst intraoperativ entschieden werden soll, ob eine rotationsfreie oder rotationsfeste Prothese gewählt wird, kann ein Prothesensystem verwendet werden, dessen Komponenten teilweise austauschbar sind (EP-B-539 654), indem eine übereinstimmende Femurkomponente und unterschiedliche Tibiakomponenten stehen. Schließlich wurde auch bereits vorgeschlagen (DE-A-2 636 816), für beide Anwendungsfälle eine rotationsfrei ausgebildete Prothese zu verwenden, bei der eine Feststellvorrichtung angebracht werden kann, die die Rotationsbewegung gewünschtenfalls ausschließt. Dies soll dadurch geschehen, daß nach der Implantation ein Loch durch den Knochen und das Rotationslager gebohrt wird, in das ein Feststellstift eingeführt wird, der die Drehung des Rotationslagers verhindert. Dies ist offensichtlich. undurchführbar. Statt dessen kann auch ein Feststellstift ausschließlich innerhalb der Prothese durch ein Rotationsaxiallager geführt werden und dessen Drehung verhindern. Es ist nicht bekannt geworden, daß dieser Vorschlag von der Praxis jemals aufgegriffen worden wäre. In der Tat stehen dem zahlreiche praktische Schwierigkeiten entgegen. Eine Rolle mag auch gespielt haben, daß im Vergleich mit einer Prothese, die ausschließlich ein Beugescharnier enthält, es verhältnismäßig aufwendig ist, eine zusätzlich mit einem Rotationslager versehene Prothese zu verwenden und dies anschließend zu arretieren.

Dennoch schließt sich die Erfindung in dem Bestreben, eine Prothese zu beschaffen, die intraoperativ eine leichte Entscheidung zwischen der rotationsfreien und der rotationsfesten Version erlaubt, an den letzteren Vorschlag an und macht ihn durch die Merkmale des Anspruchs 1 praxisgeeignet. Demnach ist der Zwischenteil, der die Femurkomponente mit der Tibiakomponente verbindet und mit der Tibiakomponente das Rotationslager bildet, mit einem Vorsprung versehen, der in eine passende Ausnehmung der Tibiakomponente eingreift.

Dieser Gedanke wird vorteilhafterweise dadurch umgesetzt, daß das Prothesensystem einen wahlweise einsetzbaren Zwischenteil (oder Komponente des Zwischenteils) mit und ohne Vorsprung umfaßt, wobei dieser Vorsprung bleibend mit dem Zwischenteil (bzw. der Komponente des Zwischenteils) verbunden ist. Dem Arzt wird dadurch die Möglichkeit gegeben, nach der Implantation sowohl der Femur- als auch der Tibiakomponente durch alternative Auswahl des den Vorsprung tragenden Teils sich für die eine oder andere Prothesenversion zu entscheiden und ggf. auch beide Versionen auszuprobieren. Statt dessen besteht auch die Möglichkeit, den Zwischenteil (bzw. dessen Komponente) mit einer Verbindungseinrichtung zum wahlweisen Verbinden mit dem Vorsprung auszurüsten.

Wenn die Tibiakomponente eine Lagerplatte mit wenigstens einer Vertiefung zur Aufnahme eines Tibiaplateaus aufweist, wird der Vorsprung zweckmäßigerweise zum Zusammenwirken mit dieser Vertiefung ausgebildet. Da diese Vertiefung großräumig ausgebildet ist, erlaubt sie eine sehr stabile Rotationsverbindung. Dabei ist die Tibiakomponente ohne jegliche Änderung in beiden Anwendungsversionen der Prothese verwendbar. Der Vorsprung wird bei dieser Ausführung zweckmäßigerweise von einer in die Vertiefung passenden Platte gebildet, die in sich und/oder mit den Begrenzungen der Vertiefung Aussparungen zur Aufnahme von Teilen eines Tibiaplateaus bildet.

Die Erfindung wird im folgenden näher unter Bezugnahme auf die Zeichnung erläutert, die ein vorteilhaftes Ausführungsbeispiel veranschaulicht. Darin zeigen:
- Fig. 1: eine Dorsalansicht der Prothese,
- Fig. 2: eine perspektivische Darstellung der Tibiakomponente mit dem Zwischenteil,
- Fig. 3: eine perspektivische Oberansicht des Tibiaplateaus,
- Fig. 4: eine perspektivische Ansicht der Tragplatte der Tibiakomponente,
- Fig. 5: eine perspektivische Unteransicht eines normalen Tibiaplateaus,
- Fig. 6: eine perspektivische Ansicht eines normalen Zwischenteils,
- Fig. 7: eine perspektivische Ansicht eines rotations-hindernden Zwischenteils,
- Fig. 8: eine perspektivische Ansicht der Tibiakomponente mit eingesetztem rotationshemmenden Zwischenteil und
- Fig. 9: eine perspektivische Unteransicht eines in Verbindung mit einem rotationshindernden Zwischenteil zu verwendenden Tibiaplateaus.

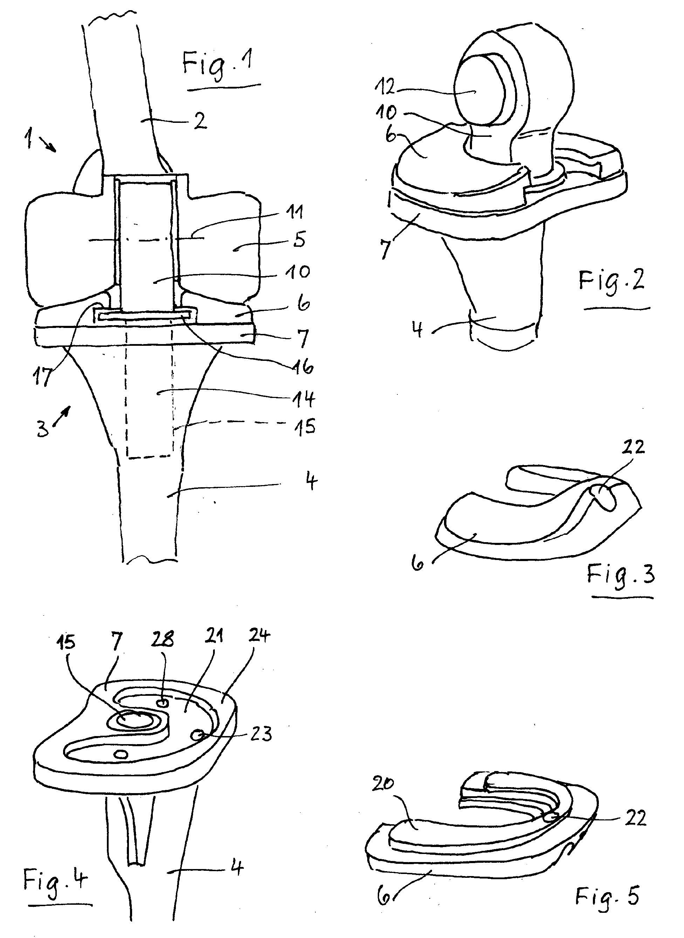

Die Prothese weist eine femorale Komponente 1 mit einem zur Implantation im Oberschenkelknochen bestimmten Dorn 2 und eine Tibiakomponente mit einem zur Implantation im Schienbein bestimmten Dorn 4 auf. Die Femurkomponente trägt flügelartige Kufen 5, die auf einem gleitgünstigen Tibiaplateau 6 aufliegen, das von einer Lagerplatte 7 der Unterschenkelkomponente getragen wird. Zur Stabilisierung der beiden Komponenten gegeneinander ist ein Zwischenstück 10 vorgesehen, das mit der Oberschenkelkomponente ein in Fig. 1 durch eine Achslinie 11 angedeutetes Scharnier bildet. In Fig. 2 erkennt man einen aus dem Zwischenstück 10 herausragenden Achsstumpf 12, der mit nicht dargestellten Lagerbohrungen in der Femurkomponente 1 zusammenwirkt. Er sitzt in einer Bohrung 13 des Zwischenteils.

Ferner bildet das Zwischenstück 10 mit der Tibiakomponente ein Rotationslager, nämlich durch den mit dem Zwischenteil einstückig verbundenen Zapfen 14, der in einer Lagerbohrung 15 der Tibiakomponente gelagert ist. Die Lagerbohrung 15 verläuft im wesentlichen parallel zu der durch den Dorn 4 an der Prothese erkennbaren Schienbeinrichtung. Das Zwischenstück 10 beschränkt somit die Relativbewegung zwischen der Femur- und Tibiakomponente auf die Beugebewegung um die Scharnierachse 11 und die Rotationsbewegung um die Achse des Rotationslagers 14, 15. Das Zwischenstück 10 kann außerdem mit einem Kragen 16 ausgestattet sein, der im Zusammenwirken mit einer Stufe 17 des Tibiaplateaus 6 das unerwünschte Abheben der Femurkomponente von der Tibiakomponente verhindert.

Das Tibiaplateau 6 besteht beispielsweise aus Polyethylen und ist oberseitig in bekannter Weise so geformt, daß im Falle einer Rotation die Femurkomponente gegenüber der Tibiakomponente ein wenig angehoben wird, so daß eine Rückstellkraft erzeugt wird, die ihn unter der übertragenen Last in die neutrale Mittelstellung zurückzuführen sucht.

Das Tibiaplateau 6 wird von der Lagerplatte 7 vorzugsweise vollflächig abgestützt. Es weist auf der Unterseite einen hufeisenförmigen Vorsprung 20 auf, dessen Höhe und Umriß passend zu denjenigen einer hufeisenförmigen Vertiefung 21 in der Lagerplatte 7 ausgebildet sind. Die korrekte Stellung des Tibiaplateaus auf der Lagerplatte wird durch das Zusammenwirken des Vorsprungs 20 und der Vertiefung 21 gesichert. Durch ein Schraubenloch 22 des Tibiaplateaus kann dies mittels einer nicht dargestellten Schraube und einer Gewindebohrung 23 in der Lagerplatte in der montierten Stellung gesichert werden. Die Lagerplatte 7 ist mit Gewindebohrungen 28 und die Sperrplatte 25 an entsprechenden Stellen mit Schraubenlöchern 29 versehen, die es gestatten, die Sperrplatte starr mit der Tibiakomponente zu verschrauben.

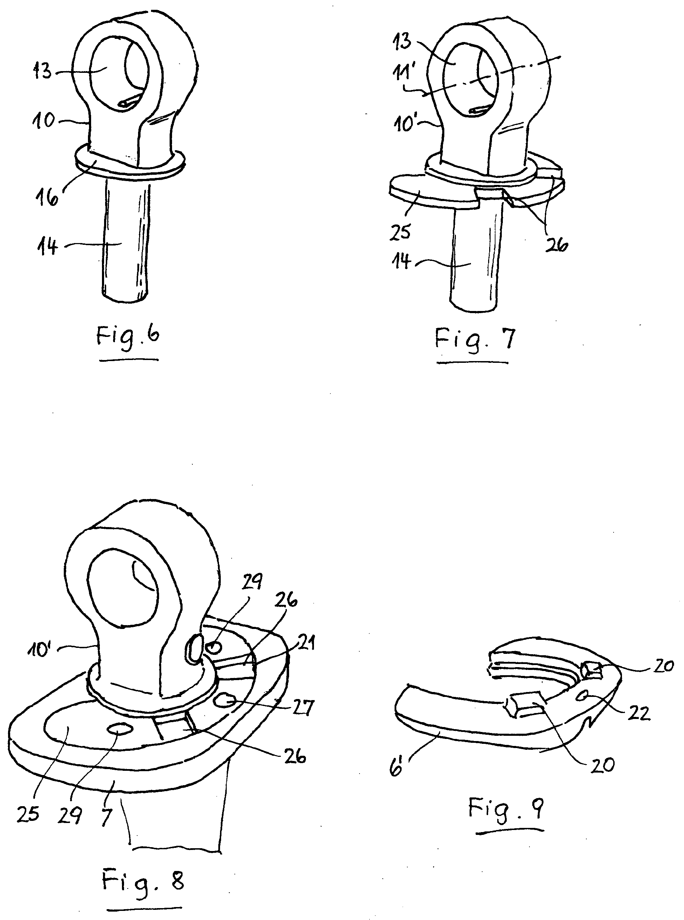

Das Zwischenstück der Normalausführung hat die in Fig. 6 gezeigte Gestalt. Alternativ ist das Zwischenstück 10' gemäß Fig. 7 verwendbar, das sich von dem normalen Zwischenstück 10 dadurch unterscheidet, daß es mit einer Sperrplatte 25 starr verbunden ist, deren Umriß genau demjenigen der Vertiefung 21 in der Lagerplatte 7 gleicht. Wenn das Zwischenstück 10' in die Tibiakomponente 3 eingesetzt ist, füllt die Sperrplatte 25 die Vertiefung 21 im wesentlichen vollständig aus. Ihre Oberfläche liegt in gleicher Höhe wie der die Vertiefung 21 umgebende Rand 24 der Lagerplatte 7. Dadurch ergibt sich eine durchgehend ebene Auflagefläche für das Tibiaplateau 6'. Um dies in der korrekten Lage zu sichern, ist es mit Vorsprüngen 20' versehen, die an die Stelle des hufeisenförmigen Vorsprungs 20 des normalen Tibiaplateaus 6 treten und passend zu Ausschnitten 26 in der Sperrplatte 25 ausgebildet sind. Im montierten Zustand greifen die Vorsprünge 20' in die Ausschnitte 26 passend ein und sichern dadurch die Lage des Tibiaplateaus. Außerdem kann es ebenso wie das normale Tibiaplateau mittels einer Schraube durch die Bohrung 22 des Plateaus und eine Bohrung 27 der Sperrplatte an der Gewindebohrung 23 der Lagerplatte 7 gesichert werden.

Die Sperrplatte 25 wird an dem Zwischenstück 10' in solcher Höhe befestigt, daß der Abstand zwischen der Sperrplatte 25 und der Scharnierachse 11' des Zwischenstücks genau der durch die Höhe der Kufen 5 und des Plateaus 6 bestimmten Lage der Scharnierachse 11 gleicht. Anders ausgedrückt sollen die durch die Kufen 5 und das Plateau 6 festgelegte Beugeachse 11 genau übereinstimmen mit der Achse 11' des Zwischenteils 10'. Dadurch soll sichergestellt werden, daß der wesentliche Teil der Lastübertragung weiterhin über den großflächigen Kontakt zwischen den Kufen 5 und dem Tibiaplateau 6 erfolgt. Falls man wünscht, daß keine Kraft über das Zwischenstück übertragen wird, wird die Sperrplatte 25 nicht starr mit dem Zwischenstück 10' verbunden, sondern axial verschiebbar, aber undrehbar. Dies kann beispielsweise dadurch geschehen, daß der Kragen 16 mit Bohrungen versehen wird und die Sperrplatte 25 zum Zapfen 4 parallele Stifte aufweist, die durch diese Bohrungen hindurchgeführt sind. Die Sperrplatte 25 kann sich dann in axialer Richtung gegenüber dem Zwischenstück 10' verschieben, ist aber über die Stifte und Bohrungen drehfest damit verbunden.

Weitere Einzelheiten der erfindungsgemäß gestalteten Prothese ergeben sich aus den gleichzeitigen Anmeldungen derselben Anmelderin unter den anwaltlichen Aktenzeichen: LINO515PEP und LINO516PEP.

## Patentansprüche

1. Knieprothese, bestehend aus einer Femurkomponente (1), einer Tibiakomponente (3) und einem diese verbindenden Zwischenstück (10, 10'), das mit der Femurkomponente ein Beugelager (11) und mit der Tibiakomponente (3) ein Rotationslager (14, 15) bildet, das gegebenenfalls gegenüber der Tibiakomponente (3) verriegelbar ist, **dadurch gekennzeichnet, daß** das Zwischenstück (10') wahlweise mit einem Vorsprung (25) versehen ist, der in eine passende Ausnehmung (25) der Tibiakomponente eingreift.

2. Knieprothese nach Anspruch 1, **dadurch gekennzeichnet, daß** wahlweise einsetzbare Zwischenstücke (10, 10') mit bzw. ohne Vorsprung (25) vorgesehen sind, wobei der Vorsprung bleibend mit einem der Zwischenstücke (10') verbunden ist.

3. Knieprothese nach Anspruch 1, **dadurch gekennzeichnet, daß** der Zwischenteil mit einer Verbindungseinrichtung zum wahlweisen Verbinden mit dem Vorsprung (25) ausgerüstet ist.

4. Knieprothese nach einem der Ansprüche 1 bis 3, **dadurch gekennzeichnet, daß** die Tibiakomponente (3) eine Lagerplatte (7) mit wenigstens einer Vertiefung (25) zur Aufnahme eines Tibiaplateaus (6) oder Teilen desselben aufweist und der Vorsprung (25) zum Zusammenwirken mit dieser Vertiefung (21) ausgebildet ist.

5. Knieprothese nach Anspruch 4, **dadurch gekennzeichnet, daß** der Vorsprung (25) von einer in die Vertiefung (21) passenden Platte gebildet ist, die in sich und/oder mit den Begrenzungen der Vertiefung (21) Aussparungen (26) zur Aufnahme von Teilen (20') eines Tibiaplateaus (6') bildet.
